# EUROPEAN PATENT SPECIFICATION

(11) **EP 3 826 630 B1**
(45) Date of publication and mention of the grant of the patent: **29.06.2022**
(21) Application number: 20842962.1
(22) Date of filing: 21.07.2020
(51) Int. Cl.: A61K 31/05, A61K 31/192, A61K 31/352, A61K 35/612, A61K 9/48, C07D 311/80, A61K 36/185

(54) **CANNABINOIDS COMPOSITIONS WITH POLYUNSATURATED FATTY ACID MONOGLYCERIDES AND USE THEREOF**
CANNABINOIDE ZUSAMMENSETZUNGEN MIT MEHRFACH UNGESÄTTIGTEN FETTSÄUREMONOGLYCERIDEN UND VERWENDUNG DAVON
COMPOSITIONS DE CANNABINOÏDES AVEC DES MONOGLYCÉRIDES D'ACIDES GRAS POLYINSATURÉS ET UTILISATION DE CELLES-CI

(30) Priority: 21.07.2019 US 201916517607; 14.08.2019 US 201962886400 P; 23.06.2020 US 202016910055
(43) Date of publication of application: 02.06.2021
(73) Proprietor: SCF Pharma Inc., Sainte-Luce, QC G0K 1P0 (CA)
(72) Inventor: FORTIN, Samuel C., Québec G0K 1P0 (CA)
(74) Representative: Icosa
(86) International application number: PCT/CA2020/051007
(87) International publication number: WO 2021/012046

(56) References cited:
- WO-A1-2008/113177
- WO-A1-2019/153073
- Shao Xianrong, Bor Gizem, Al-Hosayni Sabah, Salentinig Stefan, Yaghmur Anan: "Structural characterization of self-assemblies of new omega-3 lipids: docosahexaenoic acid and docosapentaenoic acid monoglycerides", PHYSICAL CHEMISTRY CHEMICAL PHYSICS, vol. 20, no. 37, 1 January 2018 (2018-01-01), pages 23928-23941, XP055784415, ISSN: 1463-9076, DOI: 10.1039/C8CP04256J
- Zgair Atheer, Lee Jong Bong, Wong Jonathan C. M., Taha Dhiaa A., Aram Jehan, Di Virgilio Daisy, Mcarthur Joshua W., Cheng Yu-Kit,: "Oral administration of cannabis with lipids leads to high levels of cannabinoids in the intestinal lymphatic system and prominent immunomodulation", Scientific Reports, vol. 7, no. 1, 1 December 2017 (2017-12-01), XP055784416, DOI: 10.1038/s41598-017-15026-z

## Description

### FIELD OF THE DISCLOSURE

The present document relates to the field of organic chemistry. More particularly, it relates to polyunsaturated fatty acid monoglyceride combinations with cannabinoids thereof.

### BACKGROUND OF THE DISCLOSURE

Traditionally, cannabis was consumed by smoking the dry flowers or the resin of the plant. With the recent evidence of health benefit of some cannabinoids found in the flowers or resin, like cannabidiol (CBD) or cannabidiolic acid (CBDA), *per os* formulations was developed. Lipids formulation of cannabinoids gains in popularity since Zgair shows that oral coadministration of cannabinoids with lipids can substantially increase their intestinal lymphatic transport (Zgair, A., et al., Oral administration of cannabis with lipids leads to high levels of cannabinoids in the intestinal lymphatic system and prominent immunomodulation. Scientific Reports, 2017. 7(1): p. 14542).

### SUMMARY OF THE DISCLOSURE

There is provided a composition comprising at least one compound chosen from compound of formula (I), compound of formula (II), compound of formula (III) and compound of formula (IV): 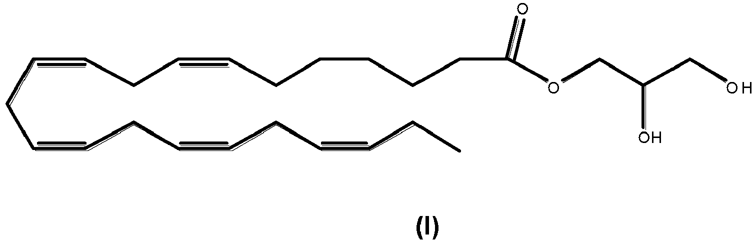 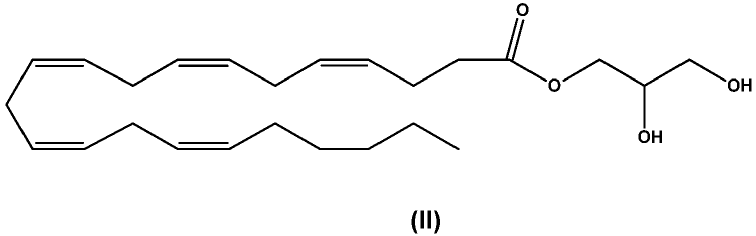 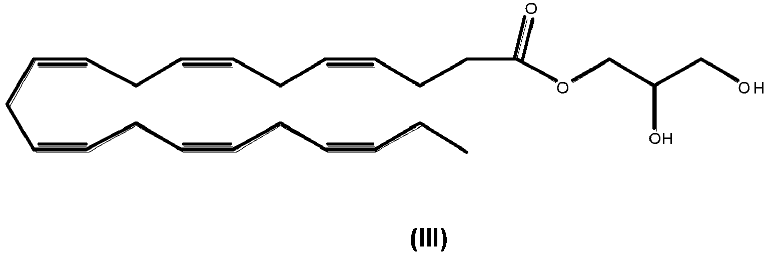 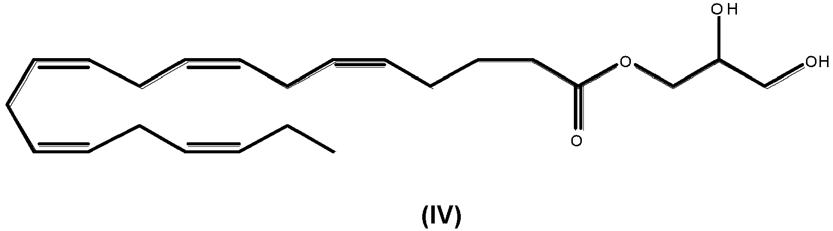
cannabidiol (CBD) in a concentration about 0.1% to about 75% by weight, based on the total weight of the composition;
tetrahydrocannabinol (THC) in a concentration less than 0.3% by weight, based on the total weight of the composition; and
at least one lipid.

There is also provided at least one compound chosen from compound of formula (I), compound of formula (II), compound of formula (III) and compound of formula (IV); and cannabidiol (CBD), for use in increasing plasma cannabidiol (CBD) concentration of a subject.

There is also provided a formulation comprising:
a shell; and
a fill material within the shell, the fill material comprising the composition as previously defined.

### BRIEF DESCRIPTION OF THE FIGURES

Further features and advantages will become more readily apparent from the following description of specific embodiments as illustrated by way of examples in the appended figures wherein:
Fig. 1 represents a comparative human absorption cross-over study of two different compositions of cannabidiol (CBD) which are medium chain triglyceride (MCT) and a composition comprising compound of formula (IV).
Fig. 2 represents a human absorption study of a composition of cannabidiolic acid (CBDA) in compound of formula (IV).
Fig. 3 represents a human absorption study of a composition of tetrahydrocannabinol (THC) in compound of formula (IV).
Fig. 4 represents a human absorption study of a composition containing a cannabinoids extract from cannabis in of compound of formula (IV) and EPA fish oil.
Fig. 5 represents the hydroxyl metabolite of CBDA in plasma following oral administration of a composition containing a cannabinoids extract from cannabis in of compound of formula (IV) and EPA fish oil.
Fig. 6 represents the carboxylic acid metabolite of CBDA in plasma following oral administration of a composition containing a cannabinoids extract from cannabis in of compound of formula (IV) and EPA fish oil.

### DETAILLED DESCRIPTION OF THE DISCLOSURE

Further features and advantages of the previously-mentioned compounds will become more readily apparent from the following description of non-limiting examples.

The term "lipid" as used herein refers to as any fat-soluble (lipophilic), molecules, such as fats, fat-like substances, oils (such as animal oil, marine oil, krill oil, fish oil or vegetable oil), waxes, sterols (such as cholesterol, ergosterol, sitosterol, stigmasterol, fat-soluble vitamins (such as vitamins A, D, E and K), fatty acids, oxidized fatty acid (such as lipoxin, specialized pro-resolving mediators or epoxydes), fatty acids esters thereof, and various derivatives thereof such as monoglycerides, diglycerides, triglycerides, phospholipids, glycolipids, and cerebrosides and pharmaceutically acceptable salts thereof. For example, the lipid can be natural or synthetic.

The term "cannabinoid" as used herein refers to at least one compound chosen from THC (Tetrahydrocannabinol), THCA (Tetrahydrocannabinolic acid), CBD (Cannabidiol),CBDA (Cannabidiolic Acid), CBN (Cannabinol), CBG (Cannabigerol), CBC (Cannabichromene), CBL (Cannabicyclol), CBV (Cannabivarin), THCV (Tetrahydrocannabivarin), CBDV, (Cannabidivarin), CBCV (Cannabichromevarin), CBGV (Cannabigerovarin), CBGM, (Cannabigerol Monomethyl Ether), CBE (Cannabielsoin), CBT (Cannabicitran) and mixtures thereof.

The term "cannabinoid extract" as used herein refers to a cannabis or hemp concentrate that comprises at least one cannabinoid and that was produced with the use of a solvent to separate the desirable compounds of cannabis or hemp from the rest of the plant matter. The most common solvents used can include, for example, butane, propane, ethanol, and supercritical carbon dioxide (CO₂). Extracts can be in several forms like: full spectrum extract, broad spectrum extract or an isolate. Full spectrum extract is a cannabis concentrate produced that preserves the full cannabinoid and terpene contents of the raw cannabis or hemp plant. The goal of a full spectrum extract is to maintain the complex range of desirable compounds in a cannabis plant without altering them through decarboxylation or oxidation. Broad spectrum extract is a full spectrum extract without the tetrahydrocannabinol (THC). An isolate is a purified form of cannabinoids typically in the range of about 80 to about 99.9%.

The expression "effective amount" of a compound of the present disclosure is a quantity sufficient to, when administered to the subject, including a mammal, for example a human, effect beneficial or desired results, including clinical results, and, as such, an "effective amount" depends upon the context in which it is being applied. The amount of a given compound of the present disclosure that will correspond to such an amount will vary depending upon various factors, such as the given drug or compound, the pharmaceutical formulation, the route of administration, the identity of the subject or host being treated, and the like, but can nevertheless be routinely determined by one skilled in the art.

Terms of degree such as "about" and "approximately" as used herein mean a reasonable amount of deviation of the modified term such that the end result is not significantly changed. These terms of degree should be construed as including a deviation of at least ±10% of the modified term if this deviation would not negate the meaning of the word it modifies.

The term "consisting essentially of", as used herein, is intended to specify the presence of the stated features, elements, components, groups, integers, and/or steps as well as those that do not materially affect the basic and novel characteristic(s) of features, elements, components, groups, integers, and/or steps.

For example, the subject in need thereof can be a bee, human, cat, dog, etc...

For example, the at least one compound is said compound of formula (I).

For example, the at least one compound is said compound of formula (II).

For example, the at least one compound is said compound of formula (III).

For example, the at least one compound is said compound of formula (IV).

For example, the at least one compound is said compound of formula (I), said compound of formula (III) and said compound of formula (IV).

For example, the at least one compound is said compound of formula (I) and said compound of formula (IV).

For example, the at least one compound is said compound of formula (I) and said compound of formula (III).

For example, the at least one compound is said compound of formula (III) and said compound of formula (IV).

For example, the at least one compound can be for use in combination with at least one ingredient chosen from cannabis crude extract and hemp crude extract.

For example, the at least one ingredient and said at least one compound can be for simultaneous administration.

For example, the at least one ingredient and said at least one compound can be for separate administration.

For example, the at least one compound chosen from compound of formula (I), compound of formula (II), compound of formula (III) and compound of formula (IV) can be administered in combination with at least one ingredient chosen from cannabis crude extract and hemp crude extract.

For example, the at least one ingredient and said at least one compound can be administered simultaneously.

For example, the at least one ingredient and said at least one compound can be administered separately.

For example, said at least one compound is said compound of formula (IV).

For example, the at least one lipid comprises at least one omega-3 fatty acid.

For example, the at least one lipid comprises eicosapentaenoic acid (EPA).

For example, the at least one lipid comprises eicosapentaenoic acid (EPA) ethyl ester.

For example, the at least one lipid comprises eicosapentaenoic acid (EPA) triglyceride.

For example, the at least one lipid comprises krill oil.

For example, the at least one cannabinoid can be an isolated cannabinoid.

For example, the at least one lipid can be a synthetic lipid.

For example, the composition or formulation can further comprise a synthetic fish oil.

For example, the composition or formulation can further comprise a synthetic ester of fish oil.

For example, the composition or formulation can further comprise a synthetic ethyl ester of fish oil.For example, the composition or formulation can further comprise glycerol.

For example, the composition of formulation can comprise about 0.05 % to about 30 % by weight, based on the total weight of the composition, of the at least one cannabinoid.

For example, the composition of formulation can comprise about 0.1 % to about 30 % by weight, based on the total weight of the composition, of the at least one cannabinoid.

For example, the composition or formulation can comprise about 0.2 % to about 30 %, about 0.5 % to about 25 %, about 10 % to about 25 %, about 1.0 % to about 20 %, about 2.0 % to about 20 %, about 2.0 % to about 30 %, about 2.0 % to about 20 %, about 3.0 % to about 20 %, about 5.0 % to about 20 %, about 1.0 % to about 15 %, about 1.0 % to about 10 %, about 1.0 % to about 10 %, about 2.0 % to about 10 %, about 1.0 % to about 15 %, about 2.0 % to about 12 %, about 5.0 % to about 15 %, about 5.0 % to about 10 %, about 0.05 % to about 2.0 %, about 0.1 % to about 10.0 %, about 0.1 % to about 5.0 %, about 0.1 % to about 2.0 %, about 0.1 % to about 1.0 %, about 0.1 % to about 0.8 %, about 0.1 % to about 0.6 %, about 0.1 % to about 0.4 %, about 0.1 % to about 0.3 %, about 0.1 % to about 0.2 %, about 0.05 % to about 50 %, about 0.05 % to about 75 %, about 30 % to about 60 %, about 10 % to about 90 %, about 10 % to about 85 %, about 0.1 % to about 75 %, or about 0.05 % to about 0.25 %, by weight, based on the total weight of the composition or formulation, of the at least one cannabinoid.

For example, the composition or formulation can comprise about 0.2 % to about 30 %, about 0.5 % to about 25 %, about 10 % to about 25 %, about 1.0 % to about 20 %, about 2.0 % to about 20 %, about 2.0 % to about 30 %, about 2.0 % to about 20 %, about 3.0 % to about 20 %, about 5.0 % to about 20 %, about 1.0 % to about 15 %, about 1.0 % to about 10 %, about 1.0 % to about 10 %, about 2.0 % to about 10 %, about 1.0 % to about 15 %, about 2.0 % to about 12 %, about 5.0 % to about 15 %, about 5.0 % to about 10 %, about 0.05 % to about 2.0 %, about 0.1 % to about 10.0 %, about 0.1 % to about 5.0 %, about 0.1 % to about 2.0 %, about 0.1 % to about 1.0 %, about 0.1 % to about 0.8 %, about 0.1 % to about 0.6 %, about 0.1 % to about 0.4 %, about 0.1 % to about 0.3 %, about 0.1 % to about 0.2 %, about 0.05 % to about 50 %, about 0.05 % to about 75 %, about 30 % to about 60 %, about 10 % to about 90 %, about 10 % to about 85 %, about 0.1 % to about 75 %, or about 0.05 % to about 0.25 %, by weight, based on the total weight of the composition or formulation, of at least one compound chosen from compound of formula (I), compound of formula (II), compound of formula (III) and compound of formula (IV).

For example, the composition or formulation can comprise about 0.2 % to about 30 %, about 0.5 % to about 25 %, about 10 % to about 25 %, about 1.0 % to about 20 %, about 2.0 % to about 20 %, about 2.0 % to about 30 %, about 2.0 % to about 20 %, about 3.0 % to about 20 %, about 5.0 % to about 20 %, about 1.0 % to about 15 %, about 1.0 % to about 10 %, about 1.0 % to about 10 %, about 2.0 % to about 10 %, about 1.0 % to about 15 %, about 2.0 % to about 12 %, about 5.0 % to about 15 %, about 5.0 % to about 10 %, about 0.05 % to about 2.0 %, about 0.1 % to about 10.0 %, about 0.1 % to about 5.0 %, about 0.1 % to about 2.0 %, about 0.1 % to about 1.0 %, about 0.1 % to about 0.8 %, about 0.1 % to about 0.6 %, about 0.1 % to about 0.4 %, about 0.1 % to about 0.3 %, about 0.1 % to about 0.2 %, about 0.05 % to about 50 %, about 0.05 % to about 75 %, about 30 % to about 60 %, about 10 % to about 90 %, about 10 % to about 85 %, about 0.1 % to about 75 %, or about 0.05 % to about 0.25 %, by weight, based on the total weight of the composition or formulation, of at least one lipid.

For example, the composition or formulation can comprise about 0.2 % to about 30 %, about 0.5 % to about 25 %, about 10 % to about 25 %, about 1.0 % to about 20 %, about 2.0 % to about 20 %, about 2.0 % to about 30 %, about 2.0 % to about 20 %, about 3.0 % to about 20 %, about 5.0 % to about 20 %, about 1.0 % to about 15 %, about 1.0 % to about 10 %, about 1.0 % to about 10 %, about 2.0 % to about 10 %, about 1.0 % to about 15 %, about 2.0 % to about 12 %, about 5.0 % to about 15 %, about 5.0 % to about 10 %, about 0.05 % to about 2.0 %, about 0.1 % to about 10.0 %, about 0.1 % to about 5.0 %, about 0.1 % to about 2.0 %, about 0.1 % to about 1.0 %, about 0.1 % to about 0.8 %, about 0.1 % to about 0.6 %, about 0.1 % to about 0.4 %, about 0.1 % to about 0.3 %, about 0.1 % to about 0.2 %, about 0.05 % to about 50 %, about 0.05 % to about 75 %, about 30 % to about 60 %, about 10 % to about 90 %, about 10 % to about 85 %, about 0.1 % to about 75 %, or about 0.05 % to about 0.25 %, by weight, based on the total weight of the composition or formulation, of the at least one synthetic SN1 monoglyceride of a vegetal or animal oil.

For example, the composition or formulation can comprise about 0.2 % to about 30 %, about 0.5 % to about 25 %, about 10 % to about 25 %, about 1.0 % to about 20 %, about 2.0 % to about 20 %, about 2.0 % to about 30 %, about 2.0 % to about 20 %, about 3.0 % to about 20 %, about 5.0 % to about 20 %, about 1.0 % to about 15 %, about 1.0 % to about 10 %, about 1.0 % to about 10 %, about 2.0 % to about 10 %, about 1.0 % to about 15 %, about 2.0 % to about 12 %, about 5.0 % to about 15 %, about 5.0 % to about 10 %, about 0.05 % to about 2.0 %, about 0.1 % to about 10.0 %, about 0.1 % to about 5.0 %, about 0.1 % to about 2.0 %, about 0.1 % to about 1.0 %, about 0.1 % to about 0.8 %, about 0.1 % to about 0.6 %, about 0.1 % to about 0.4 %, about 0.1 % to about 0.3 %, about 0.1 % to about 0.2 %, about 0.05 % to about 50 %, about 0.05 % to about 75 %, about 30 % to about 60 %, about 10 % to about 90 %, about 10 % to about 85 %, about 0.1 % to about 75 %, or about 0.05 % to about 0.25 %, by weight, based on the total weight of the composition or formulation, of the at least one synthetic diglyceride of vegetal or animal oil selected from the group consisting of SN 1,2 synthetic diglyceride and SN 1,3 synthetic diglyceride.

For example, the composition or formulation can comprise about 1 mg to about 3000 mg, about 5 mg to about 2500 mg, about 10 mg to about 2000 mg, about 75 mg to about 1100 mg, about 25 mg to about 2000 mg, about 25 mg to about 300 mg, about 50 mg to about 1500 mg, about 100 mg to about 1400 mg, about 50 mg to about 300 mg, about 10 mg to about 500 mg, about 10 mg to about 100 mg, about 10 mg to about 250 mg, about 1 mg to about 100 mg, about 1 mg to about 10 mg, about 5 mg to about 50 mg, about 5 mg to about 100 mg, about 0.1 mg to about 150 mg, about 10 mg to about 250 mg or about 100 mg to about 500 mg, of at least one compound chosen from compound of formula (I), compound of formula (II), compound of formula (III) and compound of formula (IV).

For example, the composition or formulation can comprise about 1 mg to about 3000 mg, about 5 mg to about 2500 mg, about 10 mg to about 2000 mg, about 75 mg to about 1100 mg, about 25 mg to about 2000 mg, about 25 mg to about 300 mg, about 50 mg to about 1500 mg, about 100 mg to about 1400 mg, about 50 mg to about 300 mg, about 10 mg to about 500 mg, about 10 mg to about 100 mg, about 10 mg to about 250 mg, about 1 mg to about 100 mg, about 1 mg to about 10 mg, about 5 mg to about 50 mg, about 5 mg to about 100 mg, about 0.1 mg to about 150 mg, about 10 mg to about 250 mg or about 100 mg to about 500 mg, of at least one compound cannabinoid.

For example, the composition or formulation can comprise about 1 mg to about 3000 mg, about 5 mg to about 2500 mg, about 10 mg to about 2000 mg, about 75 mg to about 1100 mg, about 25 mg to about 2000 mg, about 25 mg to about 300 mg, about 50 mg to about 1500 mg, about 100 mg to about 1400 mg, about 50 mg to about 300 mg, about 10 mg to about 500 mg, about 10 mg to about 100 mg, about 10 mg to about 250 mg, about 1 mg to about 100 mg, about 1 mg to about 10 mg, about 5 mg to about 50 mg, about 5 mg to about 100 mg, about 0.1 mg to about 150 mg, about 10 mg to about 250 mg or about 100 mg to about 500 mg, of at least one lipid.

For example, the shell is a soft shell.

For example, the shell is a soft gelatin shell.

For example, the shell is a soft starch shell.

For example, the shell is a soft carrageenan shell.

For example, the shell is a hard shell.

For example, the shell is a hard gelatin shell.

For example, the shell is a hard hypromellose shell.

For example, the shell is a hard starch shell.

For example, the shell is a hard pullulan shell

For example, as previously described, the composition or formulation can comprise the previously mentioned ingredients. For example, the composition or formulation can also consist essentially of such ingredients. For example, the composition or formulation can also consist of such ingredients.

Further features and advantages of the previously-mentioned compounds will become more readily apparent from the following description of non-limiting examples.

### EXAMPLE 1

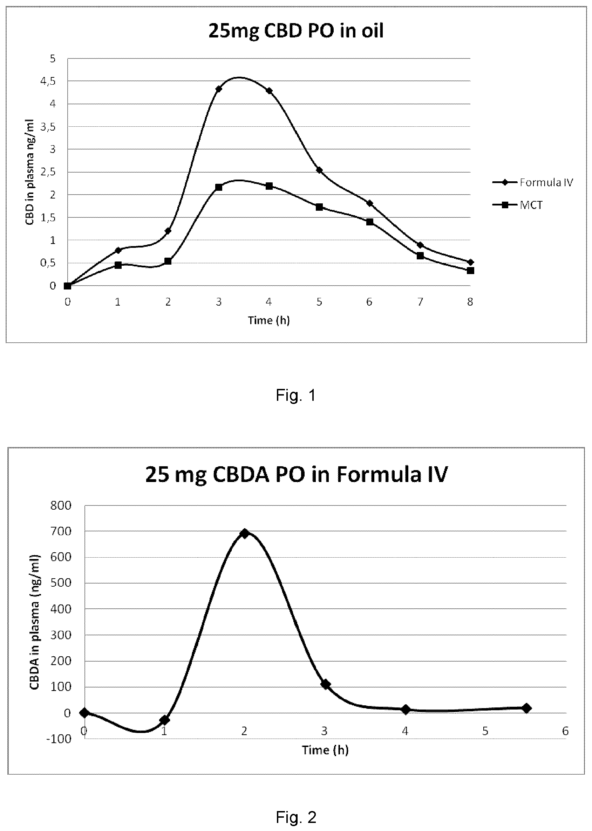

54 mg of cannabidiol (CBD) was dissolved in 2.5g of compound of formula (IV) to give a clear solution. 1.16g of the mixture (25mg CBD) was encapsulated in two (2) hard gel capsules (size 00) for absorption study. A pilot absorption study was conducted in one volunteer. The two (2) hard gel capsules were swallowed with a glass of water by the volunteer fasted for 10h. 200ul of blood was collected by a lancet in a heparinised microtube at T=0, 1, 2, 3, 4, 5, 6, 7 and 8h. The plasma was analyzed by HPLC/MS/MS to quantify the CBD. A comparative study was conducted with the same amount of CBD but with MCT oil (medium-chain triglycerides oil) instead of compound of formula (IV). Figure 1 shows the superiority of the compound of formula (IV) over the MCT oil on the absorption and bioavailability of CBD.

### EXAMPLE 2

166 mg of cannabidiolic acid (CBDA) crude extract containing 20% CBDA was dissolved in 1.33g of compound of formula (IV) to give a clear solution. 1.00g of the mixture (25mg CBDA) was encapsulated in two (2) hard gel capsules (size 00) for absorption study. A pilot absorption study was conducted in one volunteer. The two (2) hard gel capsules were swallowed with a glass of water by the volunteer fasted for 10h. 200ul of blood was collected by a lancet in a heparinised microtube at T=0, 1, 2, 3, 4, 5, 6, 7 and 8h. The plasma was analyzed by HPLC/MS/MS to quantify the CBDA. Figure **2** shows the absorption and bioavailability profile of CBDA in plasma.

### EXAMPLE 3

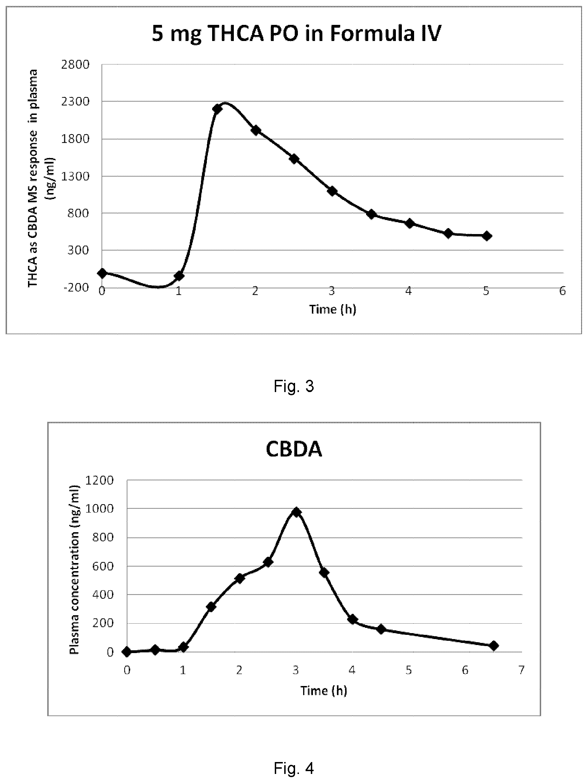

106 mg of tetrahydrocannabinolic acid (THCA) crude extract containing 20% THCA was dissolved in 4.00g of compound of formula (IV) to give a clear solution. 1.00g of the mixture (5mg THCA) was encapsulated in two (2) hard gel capsules (size 00) for absorption study. A pilot absorption study was conducted in one volunteer. The two (2) hard gel capsules were swallowed with a glass of water by the volunteer fasted for 10h. 200ul of blood was collected by a lancet in a heparinised microtube at T=0, 1, 2, 3, 4, 5, 6, 7 and 8h. The plasma was analyzed by HPLC/MS/MS to quantify the THCA (the calibration curve was made with CBDA). Figure **3** shows the absorption and bioavailability profile of THCA in plasma.

### EXAMPLE 4

### Preparation of a composition (composition 1) comprising compound IV and CBDA.

Cannabis dried flowers (7g) was extracted with ethanol (100ml) for 72h at room temperature. The flowers was filtered and the ethanol was removed under vacuum without heating to give the cannabinoid crude extract (1.9g) containing 85% CBDA and 4% THCA. The crude extract (315mg) was dissolved in a mixture of compound of formula (IV) and EPA concentrated ethyl ester fish oil (3.5g) to give composition 1.

### EXAMPLE 5

### human absorption study of composition 1 comprising compound IV and a cannabinoid crude extract containing 85% CBDA.

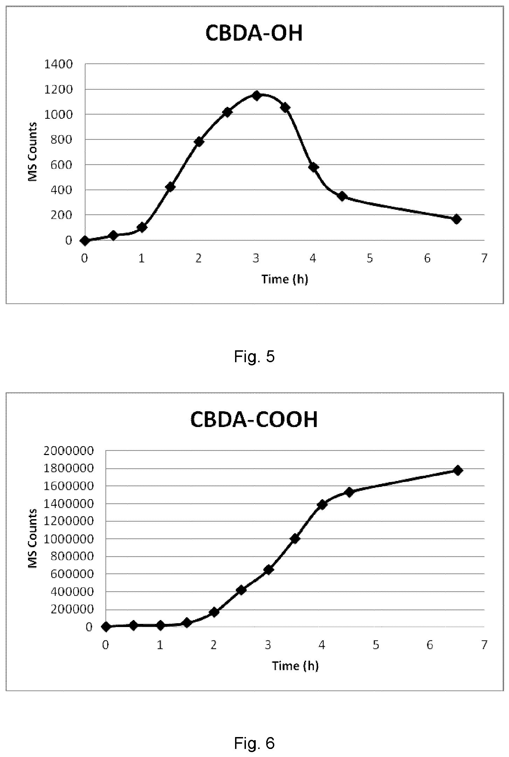

2.47g of composition 1 was encapsulated in four (4) hard gel capsules (size 00) for absorption study. A pilot absorption study was conducted in one volunteer. The four (4) hard gel capsules were swallowed with a glass of water by the volunteer fasted for 10h. 200ul of blood was collected by a lancet in a heparinised microtube at T=0, 0.5, 1, 1.5, 2, 2.5, 3, 3.5, 4, 4.5, and 6.5h. The plasma was analyzed by HPLC/MS/MS to quantify the CBDA. Figure **4** shows the absorption and bioavailability profile of CBDA in plasma. Figure **5** shows the hydroxy metabolite of CBDA in plasma and Figure **6** the carboxylic acid metabolite of CBDA in plasma.

Most of the cannabinoids lipid formulations currently on the market are with medium chain triglycerides (MCT) or seed oil. Most of the seed oil are rich in omega-6 fatty acid who are pro-inflammatory hence the need for an omega-3 rich carrier oil who can resolve inflammation. Compounds of formula I, III and IV are monoglycerides of omega-3 fatty acid and proved to have an inflammation resolution activity (Morin, C., et al., Eicosapentaenoic acid monoglyceride resolves inflammation in an ex vivo model of human peripheral blood mononuclear cell. European Journal of Pharmacology, 2017. 807: p. 205-211). In addition, the SN1 monoglyceride form is an isoform of the 2-arachidonoylglycerol (2-AG) a well-known endocannabinoids (Sugiura, T., et al., Evidence That the Cannabinoid CB1 Receptor Is a 2-Arachidonoylglycerol Receptor: STRUCTURE-ACTIVITY RELATIONSHIP OF 2-ARACHIDONOYLGLYCEROL, ETHER-LINKED ANALOGUES, AND RELATED COMPOUNDS. Journal of Biological Chemistry, 1999. 274(5): p. 2794-2801) and this close structural relationship made the compounds of formula I to IV a perfect synergistic choice for cannabinoids formulation.

Another aspect of the present disclosure is the high solubility of cannabinoids or cannabis and hemp crude extract in compound of formula IV. One of the only cannabinoid oral formulations on the market is EPIDIOLEX^{®}, a canabidiol (CBD) in corn oil at 100 mg/ml. The CBD solubility in corn oil is approx. 300 mg/ml and in a formulation containing compound of formula IV and EPA fish oil, the solubility is 400 mg/ml. This 33% increase of solubility with the ability of the formulation containing compound of formula IV and EPA fish oil to form an emulsion spontaneously in contact of water or stomach fluid or intestinal fluid, make possible an oral self emulsifying cannabinoids delivery system. In addition, this high solubility also opens the door to a high potency softgel especially for the non-psychotic cannabinoids like cannabidiol (CBD) and cannabidiolic acid (CBDA). A high potency cannabidiolic acid (CBDA) in compound of formula III can be a natural replacement of conventional NSAID drug by the COX-2 inhibition property of CBDA and pro-resolution action of compound of formula IV. In the case of cannabis or hemp flowers extract coming in from the cold extraction (without any heating) the CBD and THC will remain in their CBDA and THCA native form. In this form, CBDA is a known COX-2 inhibitor (Takeda, S., et al., Cannabidiolic Acid as a Selective Cyclooxygenase-2 Inhibitory Component in Cannabis. Drug Metabolism and Disposition, 2008. 36(9): p. 1917-1921) and THCA have no psychotropic effect who makes the cold crude extract the perfect match with the compound of formula IV.

The oral bioavailability of cannabinoids are quite low, Health Canada states an oral bioavailability of THC of only 10%. We found that when formulated in compound of formula IV, CBD is 200% more bioavailable that MCT oil at a low dose of 25 mg. To be effective as COX-2 inhibitors, the CBD or cannabinoid extract containing CBDA oral doses must be around 5mg/kg (Gallily, R., Yekhtin, Z. and Hanuǎ, L. (2015) Overcoming the Bell-Shaped Dose-Response of Cannabidiol by Using Cannabis Extract Enriched in Cannabidiol. Pharmacology & Pharmacy, 6, 75-85). At this high dose, orally administered CBD (400 mg in corn oil) give a Cmax of only 181 ng/ml in plasma (Manini, A.F., et al., Safety and pharmacokinetics of oral cannabidiol when administered concomitantly with intravenous fentanyl in humans. Journal of addiction medicine, 2015. 9(3): p. 204-210). With our formulation of a cannabinoid extract containing 170 mg of CBDA in compound of formula IV give a Cmax of 1000 ng/ml in plasma (500% increase of the Cmax for half of the dose). At our knowledge, it's the first time that a Cmax of a cannabinoids of 1000 ng/ml was reported in the scientific literature.

## Claims

1. A composition comprising:
at least one compound chosen from compound of formula (I), compound of formula (II), compound of formula (III) and compound of formula (IV): 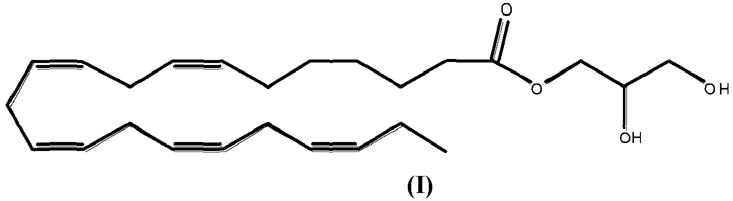 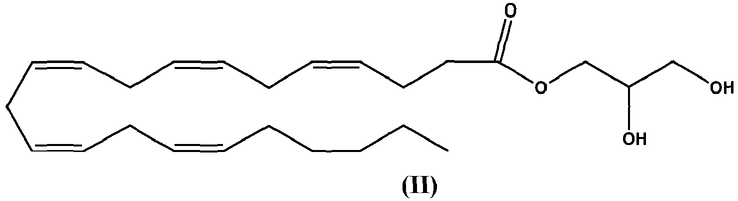 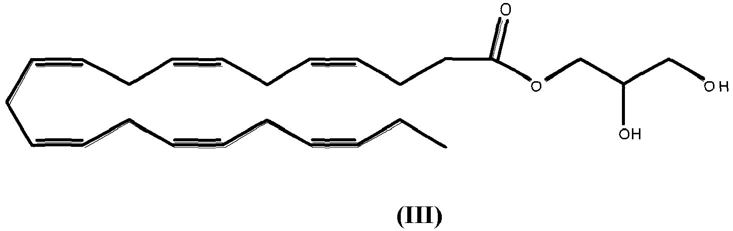 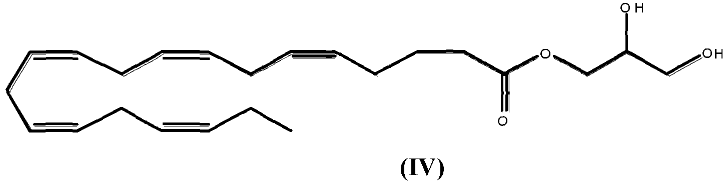 ;
cannabidiol (CBD) in a concentration about 0.1% to about 75% by weight, based on the total weight of the composition;
tetrahydrocannabinol (THC) in a concentration less than 0.3% by weight, based on the total weight of the composition; and
at least one lipid.

2. The composition of claim **1,** wherein said at least one compound is said compound of formula (IV).

3. The composition of any one of claims **1** to **2,** wherein the at least one lipid comprises at least one omega-3 fatty acid.

4. The composition of any one of claims **1** to **2,** wherein the at least one lipid comprises eicosapentaenoic acid (EPA).

5. The composition of any one of claims **1** to **2,** wherein the at least one lipid comprises eicosapentaenoic acid (EPA) ethyl ester.

6. The composition of any one of claims **1** to **2,** wherein the at least one lipid comprises eicosapentaenoic acid (EPA) triglyceride.

7. The composition of any one of claims **1** to **2,** wherein the at least one lipid comprises krill oil.

8. At least one compound chosen from compound of formula (I), compound of formula (II), compound of formula (III) and compound of formula (IV): 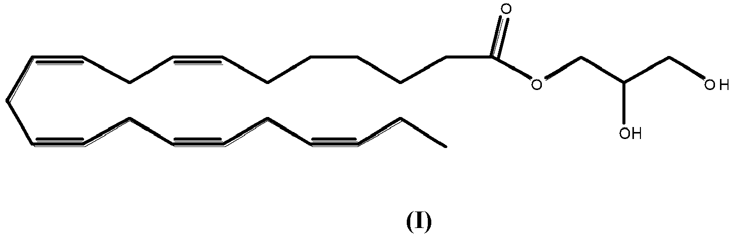 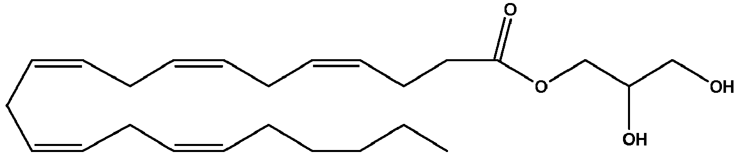 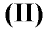 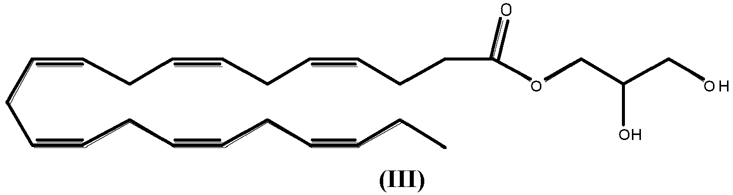 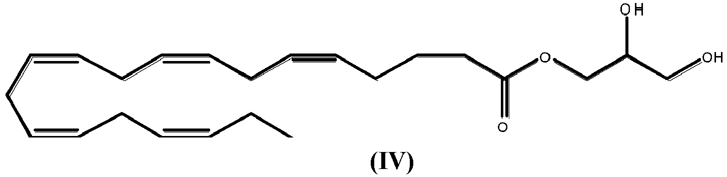 and
cannabidiol (CBD),
for use in increasing plasma cannabidiol (CBD) concentration of a subject.

9. A formulation comprising:
a shell; and
a fill material within the shell, the fill material comprising the composition of any one of claims **1** to **7.**

10. The formulation of claim **9,** wherein the shell is a soft shell.

11. The formulation of claim **9,** wherein the shell is a soft gelatin shell.

12. The formulation of claim **9,** wherein the shell is a soft carrageenan shell.

13. The formulation of claim **9,** wherein the shell is a hard shell.

14. The formulation of claim **9,** wherein the shell is a hard gelatin shell.

15. The formulation of claim **9,** wherein the shell is a hard hypromellose shell.

## Patentansprüche

1. Zusammensetzung, umfassend:
mindestens eine Verbindung, die aus einer Verbindung der Formel (I), Verbindung der Formel (II), Verbindung der Formel (III) und Verbindung der Formel (IV) ausgewählt ist: 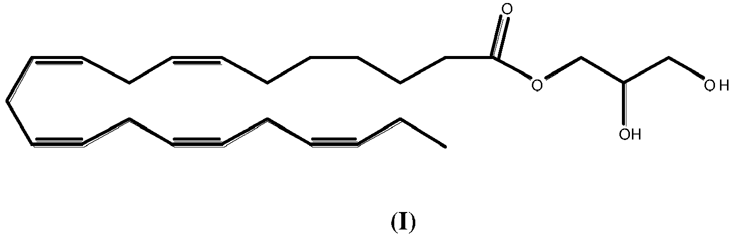 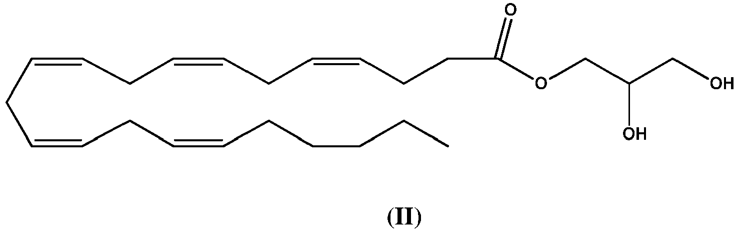 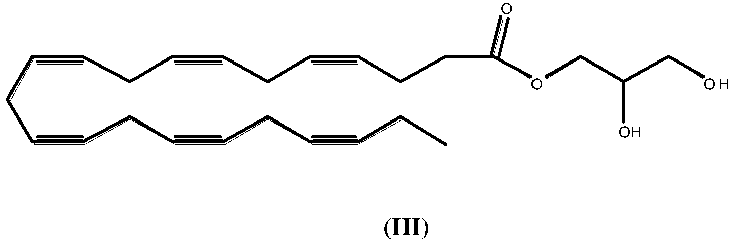 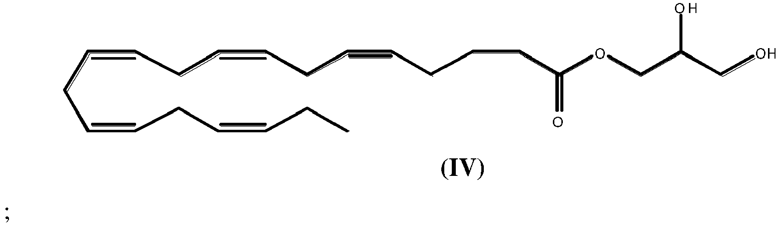
Cannabidiol (CBD) in einer Konzentration von etwa 0,1 Gew.-% bis etwa 75 Gew.-%, bezogen auf das Gesamtgewicht der Zusammensetzung;
Tetrahydrocannabinol (THC) in einer Konzentration von weniger als 0,3 Gew.-%, bezogen auf das Gesamtgewicht der Zusammensetzung; und
mindestens ein Lipid.

2. Zusammensetzung nach Anspruch **1,** wobei die mindestens eine Verbindung die Verbindung der Formel (IV) ist.

3. Zusammensetzung nach einem der Ansprüche **1** bis **2,** wobei das mindestens eine Lipid mindestens eine Omega-3-Fettsäure umfasst.

4. Zusammensetzung nach einem der Ansprüche **1** bis **2,** wobei das mindestens eine Lipid Eicosapentaensäure (EPA) umfasst.

5. Zusammensetzung nach einem der Ansprüche **1** bis **2,** wobei das mindestens eine Lipid Eicosapentaensäureethylester (EPA-Ethylester) umfasst.

6. Zusammensetzung nach einem der Ansprüche **1** bis **2,** wobei das mindestens eine Lipid Eicosapentaensäuretriglycerid (EPA-Triglycerid) umfasst.

7. Zusammensetzung nach einem der Ansprüche **1** bis **2,** wobei das mindestens eine Lipid Krillöl umfasst.

8. Mindestens eine Verbindung, die aus einer Verbindung der Formel (I), Verbindung der Formel (II), Verbindung der Formel (III) und Verbindung der Formel (IV) ausgewählt ist: 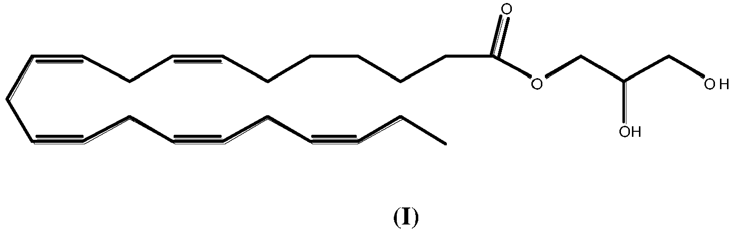 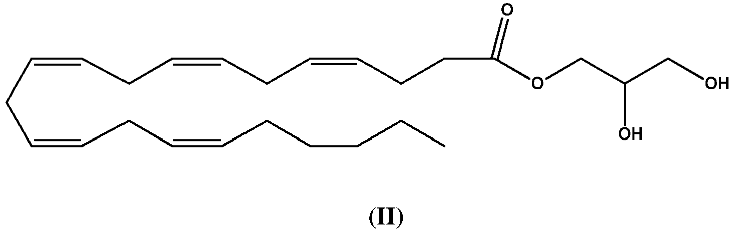 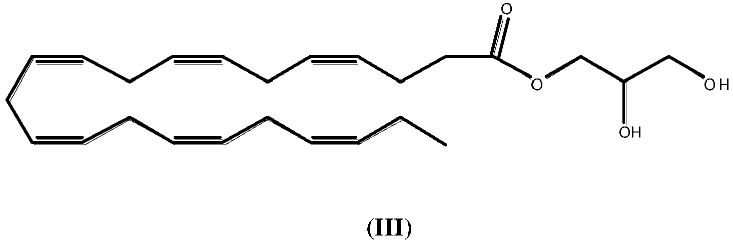 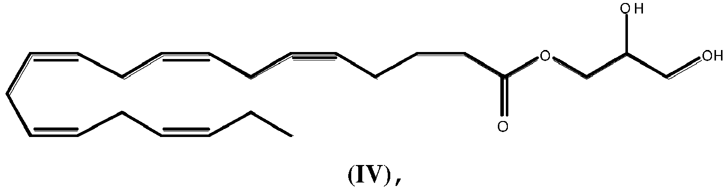 und
Cannabidiol (CBD),
zur Verwendung bei der Erhöhung der Konzentration von Cannabidiol (CBD) in Plasma eines Probanden.

9. Formulierung, umfassend:
eine Schale und
ein Füllmaterial innerhalb der Schale, wobei das Füllmaterial die Zusammensetzung nach einem der Ansprüche **1** bis **7** umfasst.

10. Formulierung nach Anspruch **9,** wobei die Schale eine weiche Schale ist.

11. Formulierung nach Anspruch **9,** wobei die Schale eine weiche Gelatineschale ist.

12. Formulierung nach Anspruch **9,** wobei die Schale eine weiche Carrageenschale ist.

13. Formulierung nach Anspruch **9,** wobei die Schale eine harte Schale ist.

14. Formulierung nach Anspruch **9,** wobei die Schale eine harte Gelatineschale ist.

15. Formulierung nach Anspruch **9,** wobei die Schale eine harte Hypromelloseschale ist.

## Revendications

1. Composition comprenant :
au moins un composé choisi parmi le composé de formule (I), le composé de formule (II), le composé de formule (III) et le composé de formule (IV) : 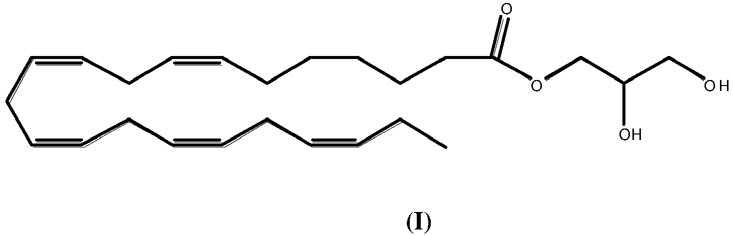 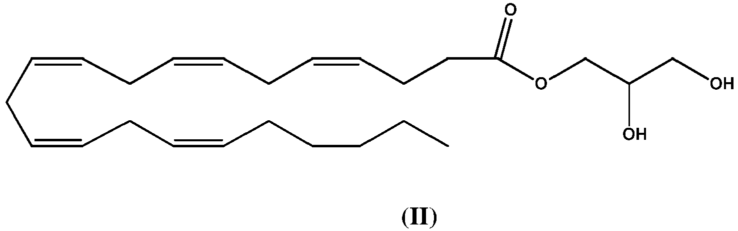 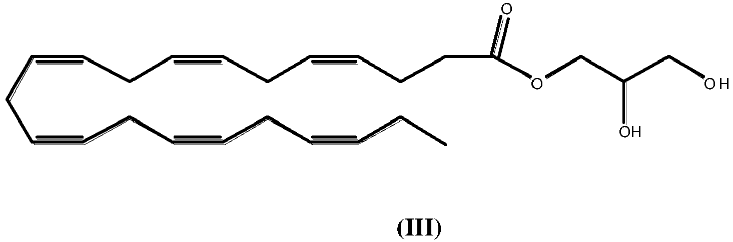 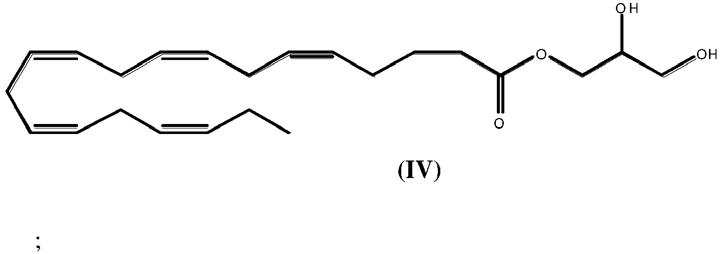
du cannabidiol (CBD) à une concentration d'environ 0,1% à environ 75% en poids, par rapport au poids total de la composition ;
du tétrahydrocannabinol (THC) à une concentration inférieure à 0,3 % en poids, par rapport au poids total de la composition ; et
au moins un lipide.

2. Composition selon la revendication **1,** dans laquelle ledit au moins un composé est ledit composé de formule (IV).

3. Composition selon l'une quelconque des revendications **1** à **2,** dans laquelle le au moins un lipide comprend au moins un acide gras oméga-3.

4. Composition selon l'une quelconque des revendications **1** à **2,** dans laquelle le au moins un lipide comprend de l'acide eicosapentaénoïque (EPA).

5. Composition selon l'une quelconque des revendications **1** à **2,** dans laquelle le au moins un lipide comprend l'ester éthylique de l'acide eicosapentaénoïque (EPA).

6. Composition selon l'une quelconque des revendications **1** à **2,** dans laquelle le au moins un lipide comprend un triglycéride d'acide eicosapentaénoïque (EPA).

7. Composition selon l'une quelconque des revendications **1** à **2,** dans laquelle le au moins un lipide comprend de l'huile de krill.

8. Au moins un composé choisi parmi le composé de formule (I), le composé de formule (II), le composé de formule (III) et le composé de formule (IV) : 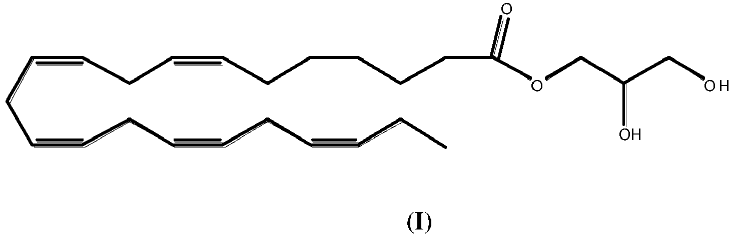 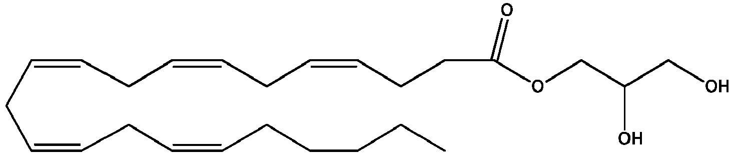 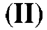 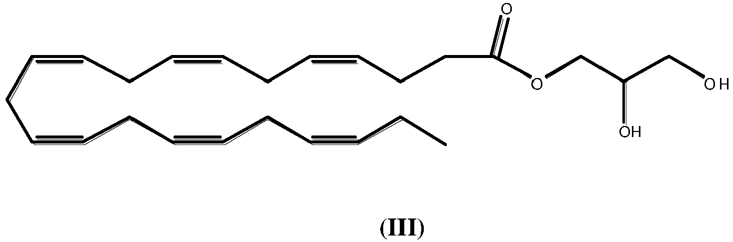 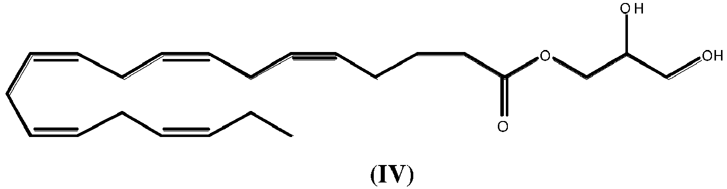 et
du cannabidiol (CBD),
pour utilisation pour augmenter la concentration plasmatique de cannabidiol (CBD) d'un sujet.

9. Une formulation comprenant :
une enveloppe ; et
un matériau de remplissage à l'intérieur de l'enveloppe, le matériau de remplissage comprenant la composition selon l'une quelconque des revendications **1** à **7.**

10. Formulation selon la revendication **9,** dans laquelle l'enveloppe est une enveloppe molle.

11. Formulation selon la revendication **9,** dans laquelle l'enveloppe est une enveloppe molle de gélatine.

12. Formulation selon la revendication **9,** dans laquelle l'enveloppe est une enveloppe molle de carraghénane.

13. Formulation selon la revendication **9,** dans laquelle l'enveloppe est une enveloppe dure.

14. Formulation selon la revendication **9,** dans laquelle l'enveloppe est une enveloppe dure de gélatine.

15. Formulation selon la revendication **9,** dans laquelle l'enveloppe est une enveloppe dure d'hypromellose.
